## Europäisches Patentamt
## European Patent Office
## Office européen des brevets

(19)

(11) Numéro de publication: **0 097 401**
**B1**

# FASCICULE DE BREVET EUROPEEN

(12)

(45) Date de publication du fascicule du brevet:
**08.10.86**

(21) Numéro de dépôt: **83200877.5**

(22) Date de dépôt: **15.06.83**

(51) Int. Cl.⁴: **G 01 V 3/11, A 61 B 17/56**

(54) **Installation pour déterminer la position d'un corps métallique dans un milieu peu conducteur de l'électricité.**

(30) Priorité: **17.06.82 LU 84209**

(43) Date de publication de la demande:
**04.01.84 Bulletin 84/1**

(45) Mention de la délivrance du brevet:
**08.10.86 Bulletin 86/41**

(84) Etats contractants désignés:
**AT BE CH DE FR GB IT LI LU NL SE**

(56) Documents cités:
**CH - A - 483 251**
**DE - A - 3 023 446**
**FR - A - 1 230 515**
**US - A - 2 228 293**
**US - A - 2 810 880**
**US - A - 3 460 528**

(73) Titulaire: **"Centre d'Etude de l'Energie Nucléaire",**
**"C.E.N.", Avenue Plasky 144, Schaerbeek**
**B-1040 Bruxelles (BE)**

(72) Inventeur: **Binard, Luc A. M. G., Avenue Grand Champ 27,**
**B - Woluwe Saint Pierre (BE)**

(74) Mandataire: **De Rycker, Rudolf, Ir. et al, Vereenigde**
**Octrooibureaux Belgie S.A. Charlottalei 48,**
**B-2018 Antwerpen (BE)**

ACTORUM AG

## Description

La présente invention est relative à une installation pour déterminer la position d'un corps métallique dans un milieu peu conducteur de l'électricité, comprenant:

- un support,
- une bobine de self-induction émettrice montée sur le support,
- deux bobines de self-induction réceptrices montées sur le support,

les bobines ayant des axes parallèles, les bobines réceptrices étant situées symétriquement par rapport à la bobine émettrice et les plans de symétrie des bobines réceptrices perpendiculaires à leurs axes coïncidant,

- une liaison à un oscillateur pour cette bobine émettrice,
- des organes de signalisation, et
- une connexion entre les bobines réceptrices et les organes de signalisation.

Une installation pour détecter le déplacement d'une masse métallique comprenant l'ensemble des éléments mentionnés ci-dessus est connue de FR-A-1 230 515.

Dans cette installation connue les plans de symétrie des bobines réceptrices perpendiculaires à leur axe et le plan de symétrie de la bobine émettrice perpendiculaire à l'axe de cette dernière coïncident sensiblement et entre les bobines réceptrices il n'y a pas de place disponible en face de la bobine émettrice.

Cette disposition relative connue de la bobine émettrice et des bobines réceptrices est acceptable lorsqu'il s'agit de détecter le déplacement d'une masse métallique mais ne se prête pas à la localisation d'un corps métallique dans une matière mauvaise conductrice de l'électricité avec une très grande précision telle que celle qui est requise lors d'opérations de corps humains et qui est, par exemple, de l'ordre d'une fraction de millimètre. En outre la disposition relative des bobines de cette installation connue est telle qu'elle ne permet pas de placer le support avec ses bobines en bonne position pour que l'asymétrie de l'objet métallique par rapport aux bobines influence d'une façon optimale la commande des organes de signalisation par les f.e.m. induites dans les bobines réceptrices.

L'invention a pour but de remédier à ces inconvénients et à cet effet

- lesdits plans du symétrie des bobines réceptrices diffèrent du plan de symétrie de la bobine émettrice perpendiculaire à son axe, et
- l'espace entre les bobines réceptrices en face de la bobine émettrice demeure libre.

Une installation dans laquelle lesdits plans de symétrie des bobines réceptrices different dudit plan de symétrie de la bobine émettrice est connue de US-A-2 228 293. Cette installation est également destinée à la détection du déplacement d'une masse métallique et ne se prête pas à la localisation précise d'un corps métallique dans une matière mauvaise conductrice de l'électricité, cette installation connue ne permettant pas à ladite matière et audit crops, d'être placés en face de la bobine émettrice entre les bobines réceptrices et ne pouvant en outre pas être connectée à un oscillateur.

Dans une forme de réalisation préférée de l'invention le support comprend un bras dont un plan de symétrie comprend l'axe de la bobine émettrice et est perpendiculaire au plan défini par les axes des bobines réceptrices, la bobine émettrice et le bras présentent un canal coaxial avec la bobine émettrice, le bras présente en outre une série de trous qui sont parallèles aux axes et qui ont leur axe dans ledit plan de symétrie, et l'oscillateur et les organes de signalisation font partie d'un dispositif électronique auquel les bobines sont reliées par des câbles.

BE-A-865 635 décrit l'introduction d'une broche dans le canal médullaire d'un os fracturé et le placement de deux groupilles transversales aux deux extrémités de la broche à travers l'os et les trous prévus à cet effet dans la broche.

Suivant ce brevet le forage de l'os pour le placement des goupilles et le placement de ces goupilles sont effectués en utilisant un gabarit solidaire de la broche.

L'expérience a démontré que le forage de l'os pour le placement de la goupille proximale et ce placement peuvent être effectués en utilisant ce gabarit solidaire de la broche, mais que ce gabarit ne peut être utilisé lors du forage de l'os pour le placement de la goupille distale et lors de ce placement que si le canal médullaire de l'os fracturé est rigoureusement droit, ce qui est rarement le cas.

Le forage de l'os pour le placement de la goupille distale et le placement de cette goupille requièrent par conséquent un autre moyen pour la localisation avec grande précision de la broche en toutes circonstances.

L'installation suivant la forme de réalisation préférée définie ci-dessus constitue un tel moyen.

Dans une forme de réalisation particulière de l'invention

- l'oscillateur est à une fréquence de l'ordre de 30 kilohertz et
- la connexion entre les bobines réceptrices et les organes de signalisation commande ces derniers en fonction du déphasage des signaux produits par les bobines réceptrices.

La combinaison de cette fréquence avec une commande des organes de signalisation en fonction du déphasage de signaux produits par les bobines réceptrices donne une localisation particulièrement fiable. Il est cependant à remarquer que l'application de cette fréquence dans une installation comprenant des bobines émettrices et d'une bobine réceptrice est connue comme telle de DE-A 3 023 446.

D'autres détails et particularités de l'invention ressortiront de la description d'une installation pour déterminer la position d'un corps métallique dans un milieu peu conducteur de l'électricité suivant l'invention, donnée ci-après à titre d'exemple non limitatif avec référence aux dessins ci-annexés.

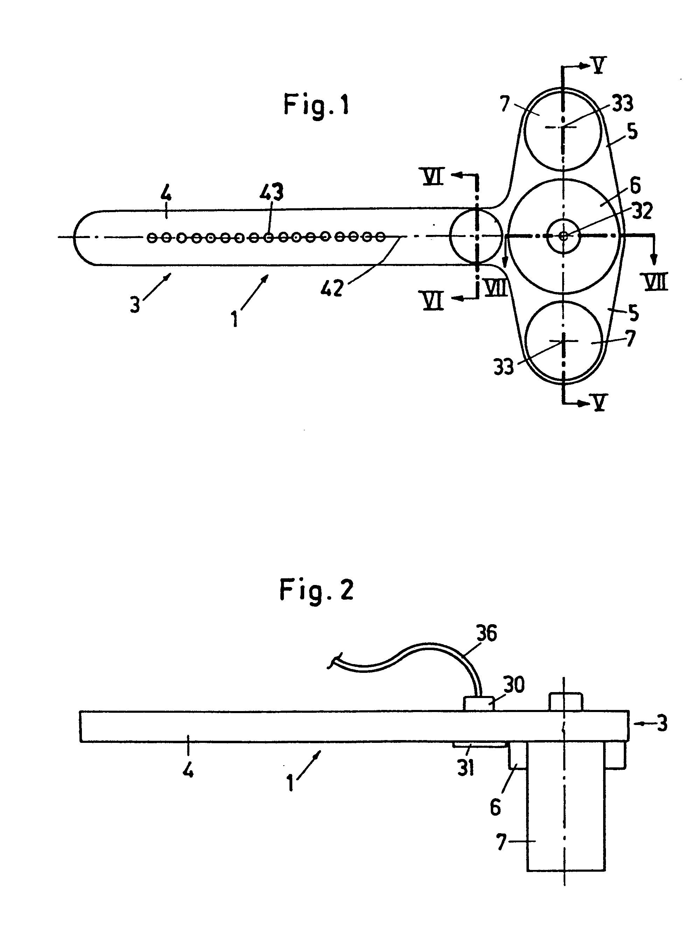

La figure 1 est une vue en plan d'une partie d'une installation pour déterminer la position d'un corps métallique dans un milieu peu conducteur de l'électricité suivant l'invention.

La figure 2 est une vue en élévation de la partie de l'installation représentée à la figure 1.

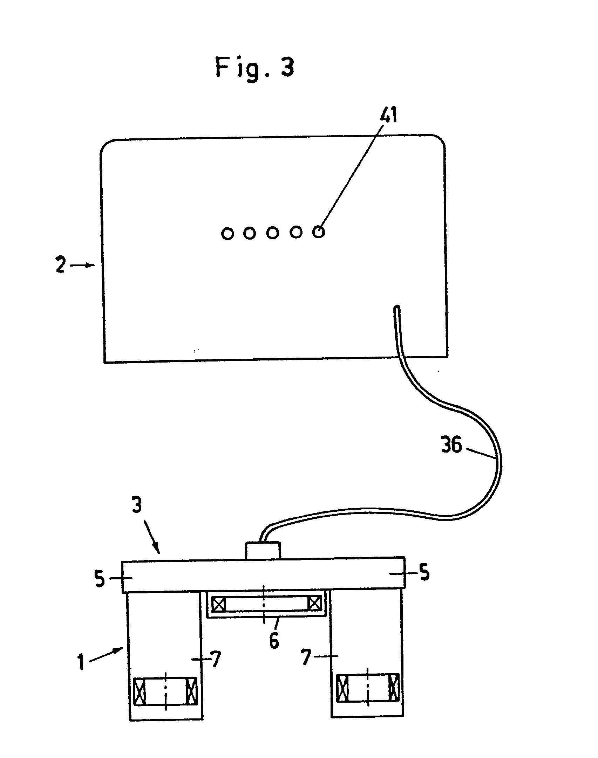

La figure 3 est une vue de côté de l'installation dont une partie est représentée aux figures 1 et 2.

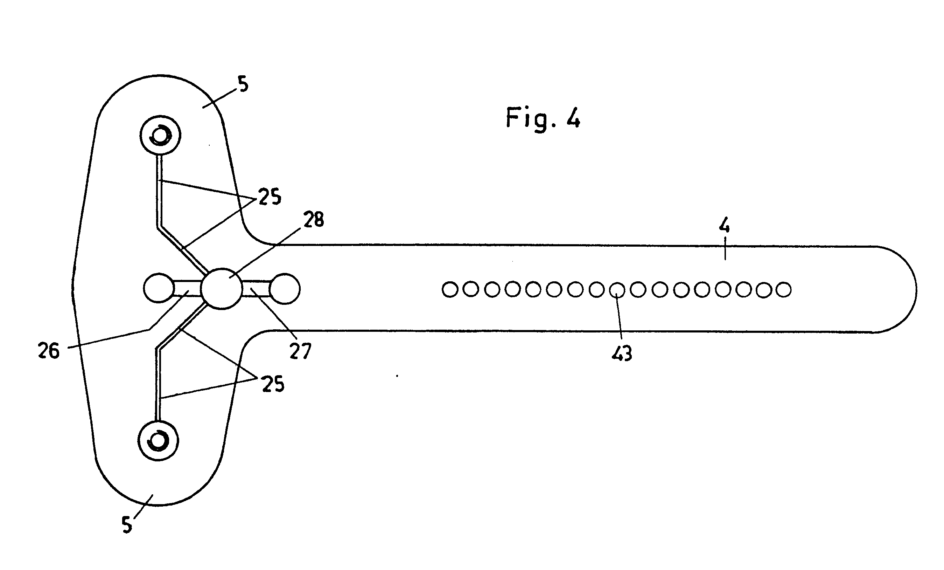

La figure 4 est une vue d'en bas d'une partie de l'installation suivant les figures sprécédentes.

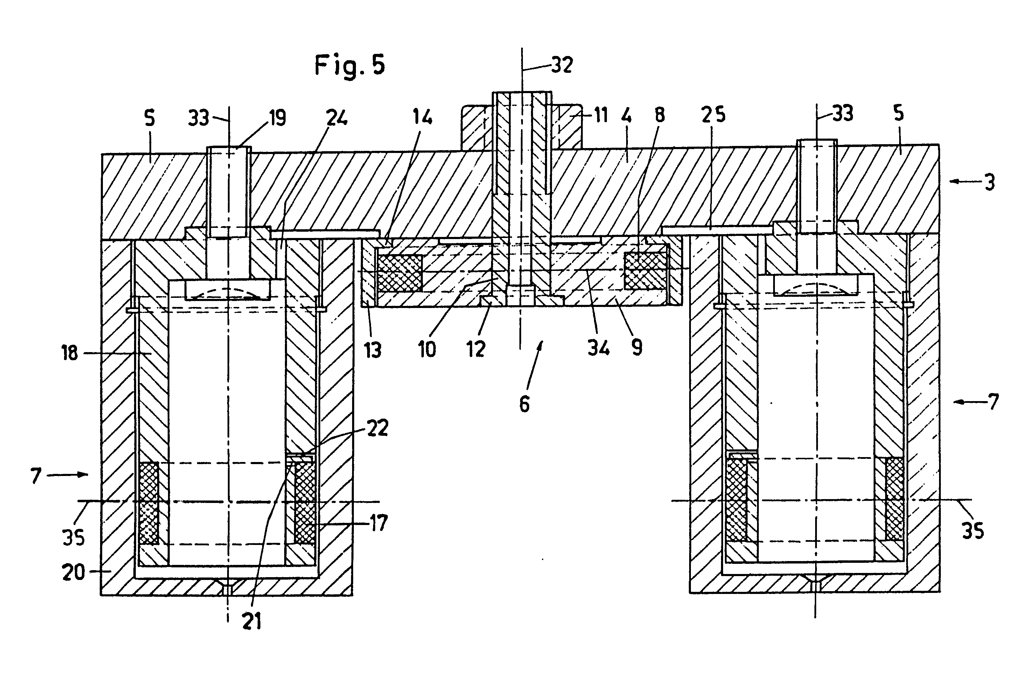

La figure 5 est une vue en coupe, à une échelle plus grande, suivant la ligne V-V de la figure 1.

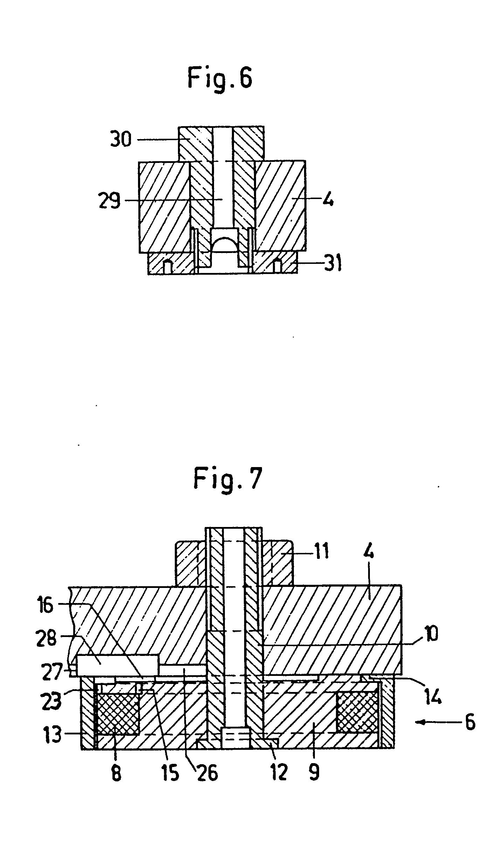

La figure 6 est une vue en coupe, à l'échelle de la figure 5, suivant la ligne VI-VI de la figure 1.

La figure 7 est une vue en coupe, à l'échelle des figures 5 et 6, suivant la ligne VII-VII de la figure 1.

La figure 8 est un diagramme synoptique de la partie électrique de l'installation suivant les figures précédentes.

Dans les différentes figures les mêmes notations de référence désignent des éléments identiques.

L'installation représentée aux figures est destinée à la localisation avec grande précision de l'extrémité d'une broche introduite dans un os entouré de tissu musculaire, de façon à pouvoir effectuer des incisions dans le tissu musculaire et à pouvoir forer dans cet os un canal transversal exactement dans l'axe du canal transversal ménagé dans cette extrémité de la broche pour l'introduction par la suite de la goupille distale.

L'introduction de la broche dans l'os et le placement de la goupille proximale sont réalisés avant l'emploi de l'installation suivant l'invention, de la façon décrite dans le brevet belge no. 965 635.

L'installation représentée aux figures se compose d'un viseur électromagnétique 1 et d'un dispositif électronique 2.

Le viseur électromagnétique comprend un support en matière plastique 3 en forme de T, composé d'un bras 4 et de deux branches 5.

Contre la face inférieure du support 3 sont montées trois bobines: une première bobine de selfinduction 6, qui est une bobine émettrice, et deux secondes bobines de self-induction 7, qui sont des bobines réceptrices.

La bobine émettrice 6 est composée d'un enroulement 8 porté par un noyau 9 en matière plastique fixé sur la face inférieure du bras 4 entre les branches 5 au moyen d'un tube 10 formant boulon et d'un écrou 11 en matière plastique. Ce tube 10 est en un métal bon conducteur de l'électricité et non magnétique tel que l'aluminium.

Le rebord 12 du tube 10 se loge dans un creux ménagé dans la face inférieure du noyau 9 et l'écrou 11 prend appui sur la face supérieure du bras 4.

Un capuchon 13 en matière plastique est pris avec son rebord 14 entre le noyau 9 et la face inférieure du bras 4. Le capuchon 13 recouvre l'enroulement 8.

Un trou 15, un passage 23 et une rainure 16 sont ménagés dans un rebord du noyau 9 pour le passage des fils (non représentés) menant à l'enroulement 8.

La rainure se prolonge dans le rebord 14 du capuchon 13.

Chacune des bobines réceptrices 7 est composée d'un enroulement 17 porté par un noyau 18 en matière plastique fixé sur la face inférieure d'une branche 5 au moyen d'un boulon 19 en matière plastique vissé dans la branche.

Un capuchon 20 en matière plastique recouvre le noyau 18, sur lequel il est vissé, ainsi que l'enroulement 17.

Dans le noyau 18 sont ménagés des trous 21, 22 et 24 pour le passage des fils (non représentés) menant à l'enroulement 17.

Les fils (non représentés) menant aux enroulements 8 et 17 sont guidés à partir des trous 24 et de la rainure 16 à travers des rainures 25, 26, et 27, et un évidement 28 vers l'entrée d'un canal 29 ménagé dans une pièce de guidage 30 en matière plastique. Cette pièce 30 est montée dans le bras 4 qu'elle traverse et dans lequel elle est maintenue par une plaque 31 vissée sur son extrémité.

L'axe 32 de la bobine émettrice 6 et les axes 33 des bobines réceptrices 7 sont situés dans un même plan.

Les plans de symétrie 35 des bobines réceptrices 7 perpendiculaires à leurs axes 33 coïncident et diffèrent du plan de symétrie 34 de la bobine émettrice 6 perpendiculaire à son axe 32.

La distance entre les plans 35 d'une part et le plan 34 d'autre part dépend du genre d'os au traitement duquel l'installation est destinée et est en général de l'ordre de 1 à 10 cm et préférentiellement de l'ordre de 6 à 7 cm.

La hauteur de l'neroulement 8, c'est-à-dire sa dimension dans le sens de l'axe 32, est de l'ordre de 1 cm. La hauteur des enroulements 17, c'est-à-dire leur dimension dans le sens des axes 33, dépend du genre d'os au traitement duquel l'installation est destinée et est en général de l'ordre de 2 à 3 cm.

C'est essentiellement grâce à la disposition relative des bobines qu'il se crée un déphasage suffisant entre les f.e.m. produites dans les bobines réceptrices 7 sous l'influence du champ électromagnétique crée par la bobine émettrice 6 alimentée en courant alternatif, lorsqu'une broche métallique est disposée asymétriquement entre les bobines 7.

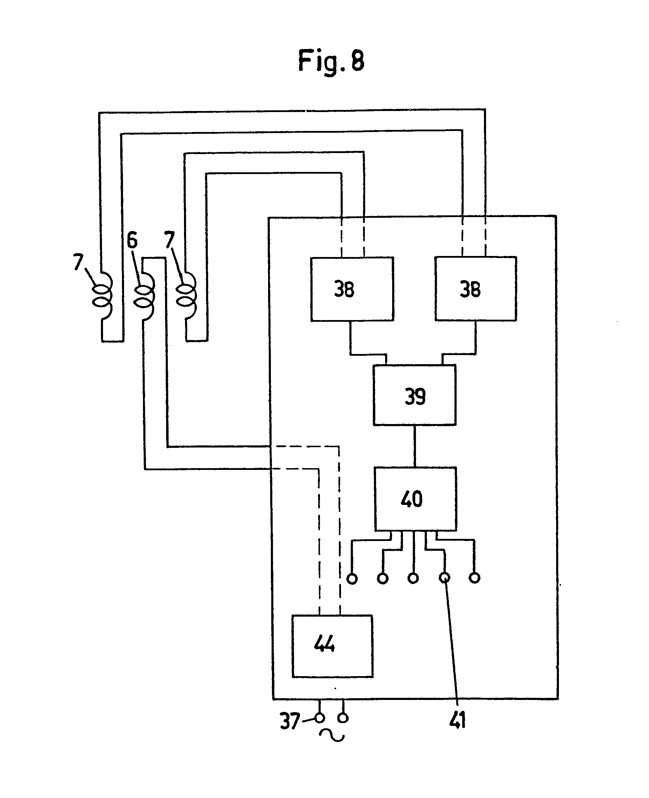

Les câbles 36 comprenant les fils menant aux enroulements 8 et 17 relient le viseur électromagnétique 1 au dispositif électronique 2 suivant le diagramme synoptique de la figure 8.

Ce dispositif est alimenté à partir du réseau 37. Il comprend un oscillateur 44 qui alimente en courant alternatif la bobine émettrice 6. Cette dernière reçoit un courant d'une fréquence de l'ordre de 30 kilohertz. Le champ électromagnétique ainsi crée induit des f.e.m. dans les bobines réceptrices 7. Ces f.e.m. sont parfaitement en phase s'il n'y a pas de métal entre les bobines 7 ou si entre ces dernières se trouve une pièce métallique disposée d'une façon parfaitement symétrique par rapport à ces bobines.

Le dispositif électronique comprend des moyens 38 d'amplification des signaux créés dans les bobines réceptrices 7, des moyens 39 de mesure de l'angle de déphasage entre ces signaux amplifiés et un moyen 40 de comparaison de cet angle de déphasage par rapport à deux seuils positifs et à deux seuils négatifs. Ce moyen de comparaison commande à son tour cinq lampes LED 41 dont celle du milieu est verte; de part et d'autre de cette dernière se trouve une lampe orange et aux extrémités sont disposées des lampes rouges. Lorsque l'angle de déphasage mesuré est inférieur aux seuils, la lampe verte du milieu s'allume. Lorsque l'angle de déphasage dépasse le seuil positif ou négatif de valeur absolue minimale, la lampe orange de gauche ou de droite s'allume selon qu'il s'agit du seuil positif ou du seuil négatif. Lorsque l'angle de déphasage dépasse le seuil positif ou négatif de valeur absolue maximale, la lampe rouge de gauche ou de droite s'allume, selon qu'il s'agit du seuil positif ou du seuil négatif.

Le plan de symétrie 42 du bras 4, comprenant l'axe 32 de la bobine émettrice 6 et perpendiculaire au plan des axes 33 des bobines réceptrices, comprend également les axes de trous 43 qui sont parallèles à ces axes 32 et 33. Ces trous 43 sont disposés à intervalles constants.

Ces trous 43 correspondent aux différentes broches qui peuvent être introduites dans un os lors d'une opération en ce sens qu'à la distance entre les axes des canaux ménagés dans les broches pour l'introduction des goupilles proximale et distale correspond la distance entre l'axe du tube 10 et l'axe d'un des trous.

Au cours d'une opération le viseur électromagnétique 1 est placé à califourchon sur le membre à opérer, de façon telle que la douille du gabarit solidaire de la broche introduite dans l'os fracturé se trouve à l'intérieur d'un trou 43. L'axe de ce trou coïncide alors avec l'axe du canal ménagé dans la broche pour la goupille proximale et les bobines réceptrices 7 se trouvent de part et d'autre du membre. Le viseur électromagnétique 1 peut être placé de cette façon grâce au fait que la distance libre entre les bobines réceptrices 7 est suffisante pour donner place au membre à opérer et est, par exemple, de l'ordre de 9 à 13 cm.

Le chirurgien sait lequel des trous doit être utilisé; il s'agit du trou dont l'axe est situé à la distance de l'axe du tube 10 qui est exactement égale à la distance entre les deux canaux qui sont ménagés dans la broche utilisée pour les goupilles proximale et distale. A côté de chaque trou est indiqué sur le bras 4 le numéro ou la longueur de la broche correspondante. Le chirurgien sait par conséquent quel trou est à utiliser pour l'opération en cours avec une broche déterminée.

Le canal du tube 10 peut alors être placé avec précision dans le prolongement du canal ménagé dans la broche pour la goupille distale.

Le tube 10 peut guider le foret pour le forage exact de l'os à hauteur de ce canal et pour l'introduction correcte de la goupille distale. Toutefois, il faut alors que le plan de symétrie 42 du bras 4 comprenne l'axe de la broche ou en d'autres mots que la broche soit à mi-distance entre les bobines réceptrices 7.

Or, il résulte de ce qui précède que si la broche, pièce métallique, est exactement à mi-distance entre les bobines réceptrices, la lampe verte s'allume. L'axe du tube 10 pointe alors le trou distal de la broche. Un écart léger dans un sens ou dans l'autre est signalé par l'allumage d'une lampe orange. Une lampe orange s'allume, par exemple, lorsque l'écart entre la broche et la ligne médiane entre les bobines réceptrices 7 est de l'ordre de 0,6 à 0,7 mm. Un écart plus prononcé est signalé par l'allumage d'une lampe rouge.

L'installation suivant l'invention permet ainsi une localisation parfaite du canal distal de la broche lors du forage de l'os pour la goupille distale et lors de l'introduction de cette dernière.

Il est bien entendu que l'installation et plus particulièrement le dispositif électronique peuvent être munis de moyens de réglage classiques.

Il doit également être entendu que l'invention n'est nullement limitée à la forme de réalisation décrite ci-avant et que bien de modifications peuvent y être apportées, notamment quant à la forme, au nombre, à la disposition et à la composition des éléments intervenant dans sa réalisation, sans sortir du cadre de la présente demande de brevet.

**Revendications**

1. Installation pour déterminer la position d'un corps métallique dans un milieu peu conducteur de l'électricité, comprenant:
   – un support (3),
   – une bobine de self-induction émettrice (6) montée sur le support (3),
   – deux bobines de self-induction réceptrices (7) montées sur le support (3),
   les bobines (6 et 7) ayant des axes (32 et 33) parallèles, les bobines réceptrices (7) étant situées symétriquement par rapport à la bobine émettrice (6) et les plans de symétrie (35) des bobines réceptrices (7) perpendiculaires à leurs axes (33) coïncidant,
   – une liaison (36) à un oscillateur (44) pour la bobine émettrice (6),
   – des organes de signalisation (41), et
   – une connexion (36, 38, 39, 40) entre les bobines réceptrices (7) et les organes de signalisation (41), caractérisée en ce que:
   – lesdits plans de symétrie (35) des bobines réceptrices (7) diffèrent du plan de symétrie (34) de la bobine émettrice (6) perpendiculaire à son axe (32) et
   – l'espace entre les bobines réceptrices (7) en face de la bobine émettrice (6) demeure libre.

2. Installation suivant la revendication précédente, caractérisée en ce que le support (3) comprend un bras (4) dont un plan de symétrie comprend l'axe (32) de la bobine émettrice (6) et est perpendiculaire au plan défini par les axes (33) des bobines réceptrices (7), la bobine émettrice (6) et le bras (4) présentant un canal coaxial avec la bobine émettrice (6), le bras (4) présentant en outre une série de trous (43) qui sont parallèles aux axes (32 et 33) et qui ont leur axe dans ledit plan de symétrie, l'oscillateur (44) et les organes de signalisation (41) faisant partie d'un dispositif électronique auquel les bobines (6 et 7) sont reliées par des câbles (36).

3. Installation suivant la revendication précédente, caractérisée en ce que les trous (43) sont disposés à intervales constants.

4. Installation suivant l'une ou l'autre des revendications 2 et 3, caractérisée en ce que le canal comprend un tube (10).

5. Installation suivant la revendication précédente, caractérisée en ce que le tube (10) est en un métal bon conducteur de l'électricité et non magnétique.

6. Installation suivant l'une ou l'autre des revendications précédentes, caractérisée en ce que
– l'oscillateur (44) est à une fréquence de l'ordre de 30 kilohertz et
– la connexion (36, 38, 39, 40) entre les bobines réceptrices (7) et les organes de signalisation (41) commande ces derniers en fonction du déphasage des signaux produits par des bobines réceptrices (7).

7. Installation suivant la revendication précédente, caractérisée en ce que les organes de signalisation (41) sont des lampes disposées en ligne, la lampe médiane indiquant un déphasage minimal.

8. Installation suivant la revendication précédente, caractérisée en ce que les lampes (41) ont une couleur différente en fonction de leur distance à partir de la lampe médiane.

9. Installation suivant l'une ou l'autre des revendications précédentes, caractérisée en ce que
– la distance entre le plan de symétrie (35) des bobines réceptrices (7) et le plan de symétrie (34) de la bobine émettrice (6) est de l'ordre de 6 à 7 cm, et
– la distance libre entre les bobines réceptrices (7) est de l'ordre de 9 à 13 cm.

**Patentansprüche**

1. Anordnung zum Auffinden der Position eines Metallstücks in einer Umgebung mit geringer elektrischer Leitfähigkeit, ausgestattet mit
– einem Träger (3);
– einer auf diesem Träger (3) angeordneten Sende-Selbstinduktionsspule (6);
– zwei auf diesem Träger (3) angeordneten Empfang-Selbstinduktionsspulen (7); wobei die Achsen (32 und 33) der vorgenannten Spulen (6 und 7) gleichlaufend sind, die beiden Empfangspulen (7) symmetrisch in bezug auf die Sendespule (6) angeordnet sind, und die senkrecht auf ihren Achsen (33) stehenden Symmetrieebenen (35) der Empfangspulen (33) zusammenfallen;

– einem Anschluss (36) an einen Oszillator (44) für die Sendespule (6);
– Signalisierungsmitteln (41); und
– einem Anschluss (36, 38, 39, 40) zwischen den vorgenannten Empfangspulen (7) und diesen Signalisierungsmitteln (41); dadurch gekennzeichnet, dass
– die vorgenannten Symmetrieebenen (35) der Empfangspulen (7) nicht mit der senkrecht auf ihrer Achse (32) stehenden Symmetrieebene (34) der Sendespule (6) zusammenfallen; und
– der gegenüber der Sendespule (6) befindliche Raum zwischen den vorgenannten Empfangspulen (7) ein freibleibender Raum ist.

2. Anordnung gemäss dem vorhergehenden Anspruch, dadurch gekennzeichnet, dass der vorgenannte Träger (3) mit einem Schenkel (4) versehen ist, wovon eine Symmetrieebene die Achse (32) der Sendespule (6) enthält und senkrecht auf der durch die Achsen (33) der Empfangsspulen (7) bestimmten Ebene steht, wobei die Sendespule (6) und der Schenkel (4) einen koachsial mit der Sendespule angeordneten Kanal aufweisen, dieser Schenkel (4) ausserdem eine Reihe mehrerer mit den Achsen (32 und 33) gleichlaufender Löcher (43) aufweist, deren Achse in der vorgenannten Symmetrieebene liegt, und der Oszillator (44) und die Signalisierungsmittel (41) zu einem elektronischen System gehören, an das die betreffenden Spulen (6 und 7) mittels Kabel (36) angeschlossen sind.

3. Anordnung gemäss dem vorhergehenden Anspruch, dadurch gekennzeichnet, dass die vorgenannten Löcher (43) gleich weit voneinander entfernt sind.

4. Anordnung gemäss irgendeinem der Ansprüche 2 und 3, dadurch gekennzeichnet, dass der vorgenannte Kanal mit einer Röhre (10) ausgestattet ist.

5. Anordnung gemäss dem vorhergehenden Anspruch, dadurch gekennzeichnet, dass die Röhre (10) aus einem nichtmagnetischen Metall guter elektrischer Leitfähigkeit hergestellt ist.

6. Anordnung gemäss irgendeinem der vorhergehenden Ansprüche, dadurch gekennzeichnet, dass
– die Schwingungsfrequenz des Oszillators (44) grössenordnungsmässig etwa 30 Kilohertz beträgt und
– die Steuerung des Signalierungsmittel (41) über den Anschluss (36, 38, 39, 40) zwischen denselben und den Empfangspulen (7) gemäss der Phasenverschiebung der durch die Empfangspulen (7) erzeugten Zeichen stattfindet.

7. Anordnung gemäss dem vorhergehenden Anspruch, dadurch gekennzeichnet, dass die vorgenannten Signalierungsmittel (41) reihenmässig angeordnete Leuchten sind, wobei die Mittelleuchte eine Mindestphasenverschiebung aufweist.

8. Anordnung gemäss dem vorhergehenden Anspruch, dadurch gekennzeichnet, dass die Farbe der vorgenannten Leuchten (41) gemäss ihrer Entfernung von der Mittelleuchte variiert.

9. Anordnung gemäss irgendeinem der vorhergehenden Ansprüche, dadurch gekennzeichnet, dass

– der Abstand zwischen der Symmetrieebene (35) der Empfangsspulen (7) und der Symmetrieebene (14) der Sendespule (6) grössenordnungsgemäss etwa 6–7 cm und

– der freie Abstand zwischen den beiden Empfangsspulen (7) grössenordnungsgemäss etwa 9–13 cm beträgt.

## Claims

1. Apparatus for determining the position of a metal body in an electrical low-conductivity medium, comprising:
   – a holder (3),
   – a transmitting inductance-coil (6) mounted on said holder (3),
   – two receiving inductance-coils (7) mounted on said holder (3), the coils (6 and 7) having axes (32 and 33) in parallel relationship, the receiving coils (7) being arranged symmetrically relative to the transmitting coil (6) and the symmetry planes (35) of the receiving coils (7) at right angle to the axes (33) thereof, coinciding with one another,
   – a connection (36) to an oscillator (44) for the transmitting coil (6),
   – signalling members (41), and
   – a connection (36, 38, 39, 40) between the receiving coils (7) and the signalling members (41), characterized in that:
   – said symmetry planes (35) of the receiving coils (7) differ from the symmetry plane (34) of the transmitting coil (6) at right angle to the axis thereof, and
   – the space between the receiving coils (7) facing the transmitting coil (6) remains free.

2. Apparatus according to the preceding claim, characterized in that the holder (3) comprises an arm (4) a symmetry plane of which includes the axis (32) of the transmitting coil (6) and lies at right angle to that plane defined by the axes (33) of the receiving coils (7), the transmitting coil (6) and the arm (4) having a duct co-axial with the transmitting coil (6), the arm further having a series of holes (43) which lie in parallel relationship with the axes (32 and 33) and the axes of which lie in said symmetry plane, the oscillator (44) and the signalling members (41) being part of an electronic device the coils (6 and 7) are connected to by cables (36).

3. Apparatus according to the preceding claim, characterized in that the holes (43) are arranged with constant spacings.

4. Apparatus according to either one of claims 2 and 3, characterized in that the duct comprises a tube (10).

5. Apparatus according to the preceding claim, characterized in that the tube (10) is made from a metal which is a good electricity conductor and non-magnetic.

6. Apparatus according to either one of the preceding claims, characterized in that
   – the oscillator (44) has a frequency in the range of 30 kHz, and
   – the connection (36, 38, 39, 40) between the receiving coils (7) and the signalling members (41) controls said latter members according to the phase displacement of those signals generated by the receiving coils (7).

7. Apparatus according to the preceding claim, characterized in that the signalling members (41) are lamps arranged along a line, the middle lamp showing a minimum phase displacement.

8. Apparatus according to the preceding claim, characterized in that the lamps (41) have a different colour depending on the spacing thereof from the middle lamp.

9. Apparatus according to any one of the preceding claims, characterized in that
   – the spacing between the symmetry plane (35) of the receiving coils (7) and the symmetry plane (34) of the transmitting coil (6) lies in the range from 6 to 7 cm, and
   – the free space between the receiving coils lies in the range from 9 to 13 cm.

# Fig.1

# Fig.2

# Fig. 3

Fig. 4

Fig. 5

## Fig.6

## Fig.7

# Fig.8